(19) Europäisches Patentamt
European Patent Office
Office européen des brevets

(11) **EP 2 233 585 A1**

(12) **EUROPEAN PATENT APPLICATION**
published in accordance with Art. 153(4) EPC

(43) Date of publication:
**29.09.2010 Bulletin 2010/39**

(51) Int Cl.:
**C12Q 1/68** (2006.01)   **C12N 15/09** (2006.01)
**C12Q 1/02** (2006.01)   **G01N 33/15** (2006.01)
**G01N 33/50** (2006.01)

(21) Application number: **08862330.1**

(22) Date of filing: **15.12.2008**

(86) International application number:
**PCT/JP2008/072786**

(87) International publication number:
**WO 2009/078385 (25.06.2009 Gazette 2009/26)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **18.12.2007   JP 2007325366**

(71) Applicants:
• **National Center for Global Health and Medicine Tokyo 162-8655 (JP)**
• **The University of Tokyo Bunkyo-Ku, Tokyo 113-0033 (JP)**
• **NATIONAL UNIVERSITY CORPORATION EHIME UNIVERSITY Ehime 790-8577 (JP)**

(72) Inventors:
• **YASUDA, Kazuki Tokyo 162-8655 (JP)**
• **KASUGA, Masato Tokyo 162-8655 (JP)**
• **KADOWAKI, Takashi Tokyo 113-0033 (JP)**
• **FURUTA, Hiroto Wakayama-shi Wakayama 641-8509 (JP)**
• **MAKINO, Hideichi Toon-chi Ehime 791-0204 (JP)**
• **IWASAKI, Naoko Tokyo 162-8666 (JP)**
• **HORIKAWA, Yukio Gifu-shi Gifu 501-1193 (JP)**
• **YAMAGATA, Kazuya Suita-shi Osaka 565-0871 (JP)**
• **OKA, Yoshitomo Sendai-shi Miyagi 980-8577 (JP)**

(74) Representative: **von Kreisler Selting Werner Deichmannhaus am Dom Bahnhofsvorplatz 1 50667 Köln (DE)**

(54) **TEST METHOD FOR TYPE-2 DIABETES USING GENE POLYMORPHISM**

(57)   The present invention relates to a method of testing for genetic susceptibility to type-2 diabetes in a subject that comprises detecting one or more polymorphisms present in the KCNQ1 gene and/or EIF2AK4 gene in a DNA-containing sample collected from the subject. The present invention permit a method of accurately, conveniently, and rapidly testing the genetic susceptibility of subjects to type-2 by targeting determinative genetic factors of genetic susceptibility to type-2 diabetes.

SEQUENCE LISTING

```
<110>  Japan Health Sciences Foundation
       National University Corporation Kobe University
       The University of Tokyo
       National University Corporation Ehime University
       National University Corporation Gifu University
<120>  Method for inspection of type 2 diabetes with nucleotide
       polymorphisms
<130>  A85267H
<160>  7
<170>  PatentIn version 3.4
<210>  1
<211>  19
<212>  RNA
<213>  Artificial sequence
<223>  siRNA for Kcnq1
<400>  1
gcgcgggacu agcaaagaa                                          19

<210>  2
<211>  19
<212>  RNA
<213>  Artificial sequence
<223>  siRNA for Kcnq1
<400>  2
gcccauuucc aucaucgac                                          19

<210>  3
<211>  19
<212>  RNA
<213>  Artificial sequence
<223>  siRNA for Kcnq1
<400>  3
gccuaaaaag ucugucaug                                          19

<210>  4
<211>  25
<212>  RNA
<213>  Artificial sequence
<223>  siRNA for eif2ak4
<400>  4
uacaacaaga caaagcugcc cgugg                                   25

<210>  5
<211>  25
<212>  RNA
<213>  Artificial sequence
<223>  siRNA for eif2ak4
<400>  5
uaagcuaauc cauccagaau cucuc                                   25

<210>  6
<211>  25
<212>  RNA
<213>  Artificial sequence
<223>  siRNA for eif2ak4
<400>  6
uuaagcuaua cuucaccagc ugagc                                   25
```

**Description**

**Cross-Reference to Related application**

This application claims benefit of priority to Japanese Patent Application No. 2007-325366 filed on December 18, 2007, which is expressly incorporated herein by reference in its entirety.

**Technical Background**

The present invention relates to a method of testing for genetic susceptibility to type-2 diabetes in subjects. More particularly, it relates to a method of testing for genetic susceptibility to type-2 diabetes in a subject that primarily comprises detecting one or more polymorphisms present in the KCNQ1 gene and/or EIF2AK4 gene in a DNA-containing sample collected from the subject. The present invention further relates to a method of screening for preventive agents or for treatment agents for type-2 diabetes, comprising the step of selecting a substance interacting either with the expression of the KCNQ1 gene or structurally or functionally with the KCNQ1 protein that is the expression product of this gene. Still further, the present invention relates to a method for screening preventive agents and treatment agents for type-2 diabetes, comprising the step of selecting a substance interacting either with the expression of the EIF2AK4 gene or structurally or functionally with the EIF2AK4 protein that is the expression product of this gene.

**Background Art**

Diabetes is one of the most common diseases in the world, and type-2 diabetes is a disease that is increasing particularly sharply among Asians and Africans. Type-2 diabetes is a disease characterized by a relative insufficiency of insulin due to impaired insulin secretion and/or insulin resistance. Genetic factors are thought to play roles in both impaired insulin secretion and insulin resistance. Genetic factors are said to play primary roles in impaired insulin secretion among Asians such as the Japanese in particular.

Patients with diabetes present symptoms such as polydipsia, polyurea, and nocturia. However, these symptoms are only produced following a prolonged period of abnormally high blood sugar levels and tend to occur naturally with age. Thus, few patients become aware that they are suffering from diabetes of their own accord in the early stages. Thus, many patients suffering from diabetes become aware that they are suffering from diabetes only with the appearance of symptoms accompanied by complications. The treatment of diabetes patients presenting such complications can often be extremely difficult. Accordingly, early detection and treatment are extremely effective in the treatment of diabetes. Genetic factors play a major role in type-2 diabetes, in particular. Thus, it is thought that not just the early discovery and treatment of type-2 diabetes, but its prevention, as well, are possible by discovering the genetic factors that are associated with the onset of type-2 diabetes, and detecting genetic susceptibility to type-2 diabetes based on these factors.

Thus far, linkage analysis employing microsatellites targeting afflicted blood relatives has been applied to many populations, both inside and outside Japan, to probe for genetic factors relating to the onset of type-2 diabetes (see Horikawa, Y. et al., (2000), Nat. Genet. 26, 163-175; Weedon, M. N. et al., (2004), Diabetes 53, 3002-3006; Mori, Y. et al., (2002), Diabetes 51, 1247-1255; and Nawata, H. et al., (2004), J. Hum. Genet. 49, 629-634, the contents of which are incorporated herein by reference). As a result, type-2 diabetes susceptibility genes such as CAPN10 on chromosome 2 and HNF4A on chromosome 20 have been identified thus far.

Although numerous candidate genes have been examined to date, no definitive gene factors have yet been obtained. Further, it is generally extremely difficult to discover genes relating to the onset of a specific disease through the above linkage analysis employing microsatellites targeting afflicted blood relatives. The above-mentioned CAPN10 and HNF4A are not effective as determinative marker genes for determining genetic susceptibility to type-2 diabetes. Accordingly, there is a need for a method of accurately, conveniently, and rapidly testing the genetic susceptibility of subjects to type-2 diabetes for the early discovery and treatment, as well as the prevention, of type-2 diabetes.

Accordingly, the present invention has for its object to provide a method for accurately, conveniently, and rapidly testing the genetic susceptibility of subjects to type-2 diabetes by targeting determinative genetic factors of genetic susceptibility to type-2 diabetes.

A further object of the present invention is to provide a method for screening preventive agents and treatment agents for type-2 diabetes comprising the step of selecting a substance interacting with the above factors or products obtained by means of these factors.

**Disclosure of the Invention**

To achieve the above-stated objects, the present inventors conducted genome-wide multistage association analysis using $10^5$ SNPs derived from Japanese single nucleotide polymorphisms (JSNP) with 1,612 patients with type-2 diabetes and 1,424 non-diabetic patients as subjects. As a result, they successfully detected 11 new SNPs that had not been reported thus far as genetic factors related to the onset of type-2 diabetes. To an unexpected degree, four SNPs in the KCNQ1 gene and one in the EIF2AK4 gene were associated with the onset of type-2 diabetes in a markedly more pronounced manner than other SNPs. The KCNQ1 gene is a gene among genes encoding a potassium ion channel protein. The abnormalities in this gene have been reported to cause ion channel disorders such as cardiac arrhythmia, epilepsy, hearing loss, and impaired muscle function (for example, see Vincent, (1998), Ann. Med. Feb; 30(1): 58-65, the contents of which are incorporated herein by reference). Additionally, the EIF2AK4 gene is a gene encoding a protein in the kinase family that phosphorylates the eukaryotic translation initiation factor 2α (EIF2α). Its expression product, also known as GCN2 kinase, phosphorylates the EIF2α protein, and through numerous cascading pathways, inhibits protein synthesis. Recently, it has also been thought to play a role in lipid synthesis (for example, see Howard C. Towlel, (2007), Cell Metabolism 5, February 2007, the contents of which are incorporated herein by reference). These reports make no mention of the KCNQ1 gene, the EIF2AK4 gene, or their genetic products playing a role in the onset of type-2 diabetes. That they play such roles was discovered by the present inventors; the present invention was devised based on this knowledge.

That is, the present invention provides a method of testing for the genetic susceptibility of a subject to type-2 diabetes that comprises detecting one or more polymorphisms present in the KCNQ1 gene in a DNA-containing sample collected from the subject.

Another aspect of the present invention provides a method of testing for the genetic susceptibility to type-2 diabetes of a subject that comprises testing for one or more polymorphisms selected from the group consisting of polymorphisms present in the KCNQ1 gene with a higher frequency of one of the alleles among some group of type-2 diabetes patients than among some group of persons who have not contracted type-2 diabetes in a DNA-containing sample collected from the subject.

Desirably, in the method of testing for the genetic susceptibility of a subject to type-2 diabetes of the present invention, the polymorphisms are comprised of polymorphisms that are exhibited at registry numbers rs151290, rs163184, rs2237895, and rs2237892 in the NCBI SNP Database, and polymorphisms in a state of linkage disequilibrium with these polymorphisms such that the linkage disequilibrium coefficient D' is 0.9 or greater.

Desirably, in the method of testing for the genetic susceptibility of a subject to type-2 diabetes of the present invention, a high genetic susceptibility to type-2 diabetes is determined to be present when the rs151290 genotype is C, the rs163184 genotype is G, the rs2237895 genotype is C, and/or the rs2237892 genotype is C.

Another aspect of the present invention provides a method of testing for the genetic susceptibility of a subject to type-2 diabetes that comprises detecting one or more polymorphisms present in the EIF2AK4 gene in a DNA-containing sample collected from the subject.

Another aspect of the present invention provides a method of testing for the genetic susceptibility of a subject to type-2 diabetes that comprises testing for one or more polymorphisms selected from the group consisting of polymorphisms present in the EIF2AK4 gene with a higher frequency of one of the alleles among some group of type-2 diabetes patients than among some group of persons who have not contracted type-2 diabetes in a DNA-containing sample collected from the subject.

Desirably, in the method of testing for the genetic susceptibility of a subject to type-2 diabetes of the present invention, the polymorphisms are comprised of the polymorphism that is exhibited at registry number rs2250402 in the NCBI SNP Database and polymorphisms in a state of linkage disequilibrium with this polymorphism such that the linkage disequilibrium coefficient D' is 0.9 or greater.

Desirably, in the method of testing for the genetic susceptibility of a subject to type-2 diabetes of the present invention, a high genetic susceptibility to type-2 diabetes is determined to be present when the rs2250402 genotype is C.

Desirably, the method of testing for the genetic susceptibility of a subject to type-2 diabetes of the present invention further comprises the detection of one or more polymorphisms selected from the group consisting of polymorphisms exhibited at registry numbers rs2307027, rs3741872, rs574628, rs2233647, rs3785233, and rs2075931 in the NCBI SNP Database and polymorphisms in a state of linkage disequilibrium with these polymorphisms such that the linkage disequilibrium coefficient D' is 0.9 or greater with a higher frequency of one of the alleles among some group of type-2 diabetes patients than among some group of persons who have not contracted type-2 diabetes.

Desirably, in the method of testing for the genetic susceptibility of a subject to type-2 diabetes of the present invention, a high genetic susceptibility to type-2 diabetes is determined to be present when the rs2307027 genotype is C, the rs3741872 genotype is C, the rs574628 genotype is G, the rs2233647 genotype is G, the rs3785233 genotype is C, and/or the rs2075931 genotype is A.

Another aspect of the present invention provides a kit for testing for genetic susceptibility to type-2 diabetes, comprising one or more nucleic acid probes and/or primers capable of detecting one or more polymorphisms present in the KCNQ1 gene and/or the EIF2AK4 gene.

Another aspect of the present invention provides a kit for testing for the genetic susceptibility to type-2 diabetes comprising one or more nucleic acid probes and/or primers capable of detecting one or more polymorphisms selected from the group consisting of polymorphisms exhibited at registry numbers rs151290, rs163184, rs2237895, rs2237892 and rs2250402 in the NCBI SNP Database, and polymorphisms in a state of linkage disequilibrium with these polymorphisms such that the linkage disequilibrium coefficient D' is 0.9 or greater.

Desirably, in the kit for testing for genetic susceptibility to type-2 diabetes of the present invention, the nucleic acid probe and/or primer is a nucleic acid probe and/or primer that is capable of detecting rs151290 with a genotype of C/A, rs163184 with a genotype of G/T, rs2237895 with a genotype of C/A, rs2237892 with a genotype of C/T, and/or rs2250402 with a genotype of C/A.

Desirably, the kit for testing for genetic susceptibility to type-2 diabetes of the present invention further comprises a nucleic acid probe and/or primer that is capable of detecting one or more polymorphisms selected from the group consisting of polymorphisms exhibited at registry numbers rs2307027, rs3741872, rs574628, rs2233647, rs3785233, and rs2075931 in the NCBI SNP Database and polymorphisms in a state of linkage disequilibrium with these polymorphisms such that the linkage disequilibrium coefficient D' is 0.9 or greater.

Desirably, in the kit for testing for genetic susceptibility to type-2 diabetes of the present invention, the nucleic acid probe and/or primer is a nucleic acid probe and/or primer that is capable of detecting rs2307027 with a genotype of C/T, rs3741872 with a genotype of C/T, rs574628 with a genotype of G/A, rs2233647 with a genotype of G/A, rs3785233 with a genotype of C/A, and/or rs2075931 with a genotype of A/G.

Another aspect of the present invention provides a method for screening for an interacting substance that comprises the steps of subjecting a cell expressing the KCNQ1 gene to the action of a test substance; detecting a change in the expression of the KCNQ1 gene or a change in the function of the KCNQ1 protein; and selecting a substance interacting with the KCNQ1 gene or the KCNQ1 protein based on this change.

Another aspect of the present invention provides a method for screening for an interacting substance that comprises the steps of subjecting a cell expressing the EIF2AK4 gene to the action of a test substance; detecting a change in the expression of the EIF2AK4 gene or a change in the function of the EIF2AK4 protein; and selecting a substance interacting with the EIF2AK4 gene or the EIF2AK4 protein based on this change.

Desirably, in the method for screening for an interactive substance of the present invention, the interacting substance is one that is employed to prevent or treat type-2 diabetes.

Based on the present invention, accurate, convenient, and rapid testing for pathosusceptibility to type-2 diabetes is possible by testing for pathosusceptibility polymorphisms in the KCNQ1 gene and/or EIF2AK4 gene (in genetic testing, blood testing, or the like). That is, the present invention permits pre-onset testing, risk testing, and early testing for type-2 diabetes. The present invention further permits the prevention and treatment of type-2 diabetes, and treatment of the cause of type-2 diabetes, through regulation of the expression and physiological activity of the KCNQ1 gene and/or the EIF2AK4 gene. This testing, prevention, and treatment increases the precision of testing for genetic susceptibility of type-2 diabetes by further targeting pathosusceptibility polymorphisms other than pathosusceptibility polymorphisms of the KCNQ1 gene and/or the EIF2AK4 gene.

The present invention permits the development of preventive and treatment agents for type-2 diabetes by screening for drugs that regulate expression of the KCNQ1 gene or the structure or function of the KCNQ1 protein. Similarly, the present invention permits the development of preventive and treatment agents for type-2 diabetes by screening for drugs that regulate the expression of the EIF2AK4 gene or the structure or function of the EIF2AK4 protein.

**Best Modes of Carrying Out the Invention**

The present invention will be described in greater detail below.
The method of testing for genetic susceptibility to type-2 diabetes of the present invention is characterized by comprising detecting one or more polymorphisms present in the KCNQ1 gene in a DNA-containing sample collected from a subject.

Another aspect of the present invention lies in that the method of testing for the genetic susceptibility to type-2 diabetes of a subject of the present invention is characterized by detecting for one or more polymorphisms selected from the group consisting of polymorphisms present in the KCNQ1 gene with a higher frequency of one of the alleles among some group of type-2 diabetes patients than among some group of persons who have not contracted type-2 diabetes in a DNA-containing sample collected from the subject.

Another aspect of the present invention lies in that the method of testing for the genetic susceptibility to type-2 diabetes of a subject of the present invention is characterized by detecting one or more polymorphisms present in the EIF2AK4 gene in a DNA-containing sample collected from the subject.

Another aspect of the present invention lies in that the method of testing for the genetic susceptibility to type-2 diabetes of a subject of the present invention is characterized by detecting for one or more polymorphisms selected from the group consisting of polymorphisms present in the EIF2AK4 gene with a higher frequency of one of the alleles among some group of type-2 diabetes patients than among some group of persons who have not contracted type-2 diabetes in a DNA-containing sample collected from the subject.

In the present specification, the term "subject," although not specifically limited, refers to all persons, including healthy persons, and is not limited to patients with type-2 diabetes or persons with a high genetic susceptibility to type-2 diabetes.

In the present specification, the term "DNA-containing sample," although not specifically limited, refers to a sample that contains DNA irrespective of DNA type or content.

In the present specification, the term "genetic susceptibility to type-2 diabetes" refers to the quality of being able to genetically induce the onset of type-2 diabetes.

In the present specification, the term "(genetic) polymorphism" refers to a change (substitution, deletion, insertion, dislocation, inversion, or the like) of one or multiple bases in genomic DNA, where the change is present with a frequency of 1 percent or greater in a population. Examples are where one base is replaced by another base (SNP), one to several tens of bases are deleted or inserted (DIP), and differences in the number of repetitions at a site where a sequence of repeating units of two to several tens of bases is present (those where the repeating unit is 2 to 4 bases are called microsatellite polymorphisms, and those where it is several to several tens of bases are called variable numbers of tandem repeats (VNTR)). Any polymorphism that satisfies the following conditions can be used in the testing method of the present invention, with SNPs being desirable.

Polymorphisms that can be utilized in the method of the present invention are not specifically limited other than that they be one or more polymorphism present in the KCNQ1 gene and/or EIF2AK4 gene, or:

(1) a polymorphism that is present in the KCNQ1 gene and/or EIF2AK4 gene, and
(2) the frequency of one of the alleles is significantly higher among some group of type-2 diabetes patients than among some group of persons who have not contracted type-2 diabetes.

In the present specification, the term "frequency of one of the alleles" refers to the extent to which a specific allele occurs in repeating fashion.

In the present specification, the phrase "some group of persons who have not contracted type-2 diabetes" refers to a group of persons who have been determined by some standard not to have developed type-2 diabetes. The phrase "some group of type-2 diabetes patients" refers to a group of persons who have been determined by some standard to have developed type-2 diabetes. So long as a group of type-2 diabetes patients and a group of persons who have not contracted type-2 diabetes satisfy the above, and are groups comprised of adequate numbers of individuals to provide results that are statistically reliable, the size (sample number), backgrounds of the individual samples (such as place of birth, age, sex, and diseases), and the like are not specifically limited. Since ethics dictate that the informed consent of persons providing samples be obtained, a group of patients who have contracted a disease other than type-2 diabetes at a medical facility or a subject group that has been diagnosed to not have contracted type-2 diabetes in a group testing at some locality is normally desirably employed as the group of persons who have not contracted type-2 diabetes.

Accordingly, the phrase "with a higher frequency of one of the alleles among some group of type-2 diabetes patients than among some group of persons who have not contracted type-2 diabetes" refers, for example, to the extent of the repeat occurrence of a specific allele being higher in a population of persons determined to have developed type-2 diabetes by some standard than in a population of persons determined not to have dveloped type-2 diabetes by some standard.

Examples of polymorphisms that are present in the KCNQ1 gene and the EIF2AK4 gene are known polymorphisms registered in the NCBI SNP Database (http://www.ncbi.nlm.nih.gov/SNP/), the JSNP Database (http://snp.ims.u-tokyo.ac.jp/), and the Applied Biosystems home page (http://www.appliedbiosystems.com/index.cfm). The contents of these home pages are specifically incorporated herein by reference. Examples are polymorphisms selected from the group consisting of polymorphisms exhibited at registry numbers rs151290, rs163184, rs2237895, rs2237892, and rs2250402 in the NCBI SNP Database and polymorphisms in a state of linkage disequilibrium with these polymorphisms such that the linkage disequilibrium coefficient D' is 0.9 or greater.

Polymorphisms exhibited at registry numbers rs151290, rs163184, rs2237895, rs2237892, and rs2250402 in the NCBI SNP Database and polymorphisms in a state of linkage disequilibrium with these polymorphisms such that the linkage disequilibrium coefficient D' is 0.9 or greater are desirable for use as polymorphisms in the testing method of the present invention.

Here, the phrase "linkage disequilibrium coefficient D'" can be obtained from the following equation by taking two SNPs, denoting the individual alleles of the first SNP as (A, a), denoting the individual alleles of the second SNP as (B, b), and denoting the various frequencies of the four haplotypes (AB, Ab, aB, ab) as $P_{AB}$, $P_{Ab}$, $P_{aB}$, and $P_{ab}$:

$$D' = (P_{AB}P_{ab} - P_{Ab}P_{aB})/Min[(P_{AB} + P_{aB})(P_{aB} + P_{ab}), (P_{AB} + P_{Ab})(P_{Ab} + P_{ab})]$$

(wherein Min[(P_{AB} + P_{aB})(P_{aB} + P_{ab}), (P_{AB} + P_{Ab})(P_{Ab} + P_{ab})] means taking the lesser value from among (P_{AB} + P_{aB}) (P_{aB} + P_{ab}) and (P_{AB} + P_{Ab})(P_{Ab} + P_{ab})).

For example, a polymorphism for which D' is 0.95 or higher is desirable, 0.99 or higher is preferable, and 1 is optimal.

Among the above polymorphisms, those with a significantly higher frequency of one of the alleles among some group of type-2 diabetes patients than among some group of persons who have not contracted type-2 diabetes can be utilized in the testing method of the present invention. In the present specification, such polymorphisms are also referred to as type-2 diabetes marker polymorphisms.

Polymorphisms exhibited at registry numbers rs151290, rs163184, rs2237895, rs2237892, and rs2250402 in the NCBI SNP Database and polymorphisms in a state of linkage disequilibrium with these polymorphisms such that the linkage disequilibrium coefficient D' is 0.9 or greater can be employed in testing in the present invention. The polymorphisms that are detected by the testing method of the present invention may be any one of these polymorphisms, or may be two or more of the same.

In the testing method of the present invention, any known method of detecting SNPs can be used to detect the polymorphism. Examples of classical detection methods are: the method of employing genomic DNA extracted from cells or the like of a subject and using a probe in the form of nucleic acid containing a base sequence of about 15 to about 500 continuous bases containing the bases at the polymorphism positions exhibited at registry numbers rs151290, rs163184, rs2237895, rs2237892, and rs2250402 in the NCBI SNP Database to conduct hybridization under correct stringent controls according to the method of Wallace et al. (see Proc. Natl. Acad. Sci. USA, 80, 278-282 (1983), the contents of which are incorporated herein by reference), for example, to detect only sequences that are completely complementary to the probe; and the method of employing a mixed probe, consisting of the above nucleic acid and the nucleic acid in which the bases at polymorphic sites in the above nucleic acid have been replaced with other bases, one of which has been labeled and the other of which has not, to conduct hybridization while gradually lowering the reaction temperature from the denaturing temperature to cause sequences that are fully complementary with one of the probes to hybridize first, thereby preventing cross-reactions with mismatched probe. Here, a radioactive isotope, enzyme, fluorophore, light-emitting substance, or the like can be employed as the labeling agent. Examples of radioactive isotopes that can be employed are [$^{125}$I], [$^{131}$I], [$^{3}$H], and [$^{14}$C]. The above enzyme is desirably a stable enzyme of great specific activity; β-galactosidase, β-glucosidase, alkaline phosphatase, peroxidase, malate dehydrogenase, and the like can be employed. Examples of fluorophores that can be employed are fluorescamine and fluorescein isothiocyanate. Examples of light-emitting substances that can be employed are luminol, luminol derivatives, luciferins, and lucigenin.

The polymorphism is desirably detected, for example, by one of the various methods described in WO 03/023063. For example, this can be conducted by the RFLP method, the PCR-SSCP method, ASO hybridization, the direct sequencing method, the ARMS method, the denaturing agent concentration gradient gel electrophoresis method, the RNaseA cleaving method, the chemical cleaving method, the DOL method, the TaqMan PCR method, the invader method, the MALDI-TOF/MS method, the TDI method, the molecular beacon method, the dynamic allele-specific hybridization method, the padlock probe method, the UCAN method, the nucleic acid hybridization method employing a DNA chip or DNA microarray, or the ECA method (see line 5, page 17 to line 20, page 28 of WO 03/023063, the contents of which are incorporated herein by reference). The TaqMan PCR method and the invader method will be described in greater detail as typical methods.

(a) The TaqMan PCR method

The TaqMan PCR method employs PCR based on Taq DNA polymerase and a fluorescence-labeled allele-specific oligonucleotide (a TaqMan probe). A oligonucleotide comprised of a continuous base sequence of about 15 to about 30 bases containing the bases of the polymorphism sites exhibited at registry numbers rs151290, rs163184, rs2237895, rs2237892, and rs2250402 in the NCBI SNP Database can be employed as the TaqMan probe, for example. The probe is labeled at its 5'-end with a fluorophore such as FAM or VIC, and at its 3'-end with a quencher (light-extinguishing substance) such as TAMRA. In that state, fluorescence is not detected because the quencher absorbs the fluorescent energy. Probe is prepared for both alleles and is desirably labeled with fluorophores of different fluorescence wavelengths (for example, FAM for one allele and VIC for the other) for comprehensive detection. The 3'-end is phosphorylated so that a PCR extension reaction does not occur from the TaqMan probe. When PCR is conducted with a primer designed to amplify a partial sequence of genomic DNA containing the region hybridizing with the TaqMan probe and Taq DNA polymerase, the TaqMan probe hybridizes with the template DNA. Although the extension reaction simultaneously occurs from the PCR primer, as the extension reaction progresses, the TaqMan probe that has hybridized is cleaved by the 5' nuclease activity of the DNA polymerase, releasing the fluorophore so as to remove the effect of the quencher and resulting in the detection of fluorescence. Amplification of the template causes the intensity of the fluorescence to increase exponentially.

(b) The invader method

In the invader method, in contrast to the TaqMan PCR method, the allele-specific oligonucleotide (allele probe) itself is not labeled. A sequence (flap) that is not complementary with the template DNA is present on the 5'-side of the base at a polymorphism site, and a complementary sequence specific to the template is present on the 3'-side. An oligonucleotide having a complementary sequence specific to the 3'-side of the polymorphism site of the template (an invader probe; the base corresponding to the polymorphism site at the 5'-end of the probe can be any base) and a FRET (fluorescence resonance energy transfer) probe characterized by both the presence of a sequence capable of assuming a hairpin structure on its 5'-end and by the presence of a sequence, running from the base pairing with the base on the 5'-end to the 3'-side once the hairpin structure has been formed, that is complementary to the flap of the allele probe, are employed in the invader method. The 5'-end of the FRET probe is fluorescence labeled (with FAM, VIC, or the like), and a quencher (TAMRA or the like) is bonded nearby. In that state (the hairpin structure), no fluorescence is detected.

When the genomic DNA serving as template is reacted with the allele probe and the invader probe, the 3'-end of the invader probe invades the polymorphism site as the three complementarily bond. A cleavase enzyme recognizing the structure at the polymorphism site is employed to cleave the single-strand portion of the allele probe (that is, the flap portion to the 5'-side of the base at the polymorphism site), at which point the flap bonds complementarily to the FRET probe and the polymorphism site of the flap invades the hairpin structure of the FRET probe. The recognition and cleavage of this structure with cleavase results in the release of the fluorophore label on the end of the FRET probe, removing the effect of the quencher and causing fluorescence to be detected. Allele probe in which the base at the polymorphism site does not match the template is not cut by the cleavase, but since the uncut allele probe is capable of hybridizing with the FRET probe, fluorescence is similarly detected. However, due to differences in reaction efficiency, the allele probe with the matching base at the polymorphism site produces a fluorescent intensity that is markedly greater than that of the nonmatching allele probe.

Normally, before reacting the three probes and the cleavase, it is desirable to amplify the DNA by PCR using a primer that is capable of amplifying the region containing the portion hybridized by the allele probe and invader probe.

As a result of testing for polymorphisms as set forth above, when a determination is made that an allele is present that is significantly more frequent among some group of type-2 diabetes patients than among some group of persons who have not contracted type-2 diabetes, particularly when the allele is determined to be homozygous, the subject can be determined to be highly genetically susceptible to type-2 diabetes. For example, when the NCBI SNP Database registry number rs151290 genotype is C, the rs163184 genotype is G, the rs2237895 genotype is C, the rs2237892 genotype is C, and/or the rs2250402 genotype is C, a determination of high genetic susceptibility to type-2 diabetes can be made.

In another aspect of the present invention, the method of testing for genetic susceptibility to type-2 diabetes of the present invention is characterized by including, in addition to one or more polymorphisms present on the KCNQ1 gene and/or EIF2AK4 gene, the detection of one or more polymorphisms selected from the group consisting of polymorphisms exhibited at registry numbers rs2307027, rs3741872, rs574628, rs2233647, rs3785233, and rs2075931 in the NCBI SNP Database and polymorphisms in a state of linkage disequilibrium with these polymorphisms such that the linkage disequilibrium coefficient D' is 0.9 or greater, with a higher frequency of one of the alleles among some group of type-2 diabetes patients than among some group of persons who have not contracted type-2 diabetes in the DNA-containing sample collected from a subject. For example, a high genetic susceptibility to type-2 diabetes can be determined to exist when the rs2307027 genotype is C, the rs3741872 genotype is C, the rs574628 genotype is G, the rs2233647 genotype is G, the rs3785233 genotype is C, and/or the rs2075931 genotype is A.

The kit of the present invention is a kit for testing for genetic susceptibility to type-2 diabetes characterized by comprising one or more nucleic acid probes and/or primers capable of detecting one or more polymorphisms present in the KCNQ1 gene and/or the EIF2AK4 gene. That is, the kit of the present invention is characterized by comprising one or more nucleic acid probes and/or primers capable of detecting one or more polymorphisms selected from the group of polymorphisms present in the KCNQ1 gene and/or the EIF2AK4 gene, with a higher frequency of one of the alleles among some group of type-2 diabetes patients than among some group of persons who have not contracted type-2 diabetes.

Specifically, so long as the nucleic acid probe employed in the kit of the present invention is nucleic acid hybridizing with genomic DNA in a region containing the base of a polymorphism site to be detected by the testing method of the present invention, is specific to a target site, and is capable of readily detecting polymorphism, the length thereof (the base length of the portion hybridizing with genomic DNA) is not specifically limited. For example, the length can be about 15 bases or greater, desirably about 15 to about 500 bases, preferably about 15 to about 200 bases, and optimally, about 15 to about 50 bases.

The probe may contain an additional sequence (that is not complementary to genomic DNA) suited to detecting polymorphism. For example, the allele probe employed in the above invader method comprises an additional sequence called a flap on the 5'-end of the base at the polymorphism site.

Further, the probe may be labeled with a suitable labeling agent such as a radioactive isotope (such as [125I], [131I], [3H], or [14C]), an enzyme (such as β-galactosidase, β-glucosidase, alkaline phosphatase, peroxidase, or malate dehydrogenase), a fluorophore (such as fluorescamine or fluorescein isothiocyanate), or a light-emitting substance (such as luminol, luminol derivatives, luciferins, or lucigenin). Alternately, a quencher (light-extinguishing substance) absorbing fluorescent energy emitted by a fluorophore (such as FAM or VIC) may be further bonded in the vicinity of the fluorophore. In such implementation forms, the fluorophore and the quencher are separated in the detection reaction and fluorescence is detected.

Desirably, the nucleic acid probe employed in the kit of the present invention contains a continuous base sequence in the form of about 15 to about 50 bases, desirably about 15 to about 200 bases, preferably about 15 to about 50 bases containing the base of a polymorphism site selected from the group consisting of the polymorphisms exhibited at registry numbers rs151290, rs163184, rs2237895, rs2237892, and rs2250402 in the NCBI SNP Database and polymorphisms in a state of linkage disequilibrium with these polymorphisms such that the linkage disequilibrium coefficient D' is 0.9 or greater. Preferably, a nucleic acid probe containing a continuous base sequence in the form of about 15 to about 500 bases, desirably about 15 to about 200 bases, preferably about 15 to about 50 bases containing the base of a polymorphism site selected from the group consisting of the polymorphisms exhibited at registry numbers rs2307027, rs3741872, rs574628, rs2233647, rs3785233, and rs2075931 in the NCBI SNP Database and polymorphisms in a state of linkage disequilibrium with these polymorphisms such that the linkage disequilibrium coefficient D' is 0.9 or greater is employed with the above nucleic acid probe.

The nucleic acid primer that is employed in the kit of the present invention may be any nucleic acid primer designed to permit the specific amplification of the region of genomic DNA containing the base of the polymorphism site to be detected in the testing method of the present invention. The primer may also containing an additional sequence (that is not complementary to genomic DNA) suited to the detection of polymorphism, such as a linker sequence.

The primer may also be labeled with a suitable labeling agent such as a radioactive isotope (such as [125I], [131I], [3H], or [14C]), an enzyme (such as β-galactosidase, β-glucosidase, alkaline phosphatase, peroxidase, or malate dehydrogenase), a fluorophore (such as fluorescamine or fluorescein isothiocyanate), or a light-emitting substance (such as luminol, luminol derivatives, luciferins, or lucigenin).

The nucleic acid probe or primer employed in the kit of the present invention may be either DNA or RNA, and may be either single-stranded or double-stranded. When double-stranded, it may be double-stranded DNA, double-stranded RNA, or a DNA/RNA hybrid. Accordingly, when a description is given of nucleic acid having a base sequence in the present specification, unless specifically stated otherwise, the meaning thereof is to be construed as including single-stranded nucleic acid containing the particular base sequence, single-stranded nucleic acid having a sequence complementary to the particular base sequence, and double-stranded nucleic acid hybrids thereof.

The probe and primer of the above nucleic acid can be synthesized by the usual methods using a DNA/RNA automatic synthesizer based on information on the base sequence indicated in registry number rs151290, rs163184, rs2237895, rs2237892, rs2250402, rs2307027, rs3741872, rs574628, rs2233647, rs3785233, or rs2075931 in the NCBI SNP Database, for example.

The one or more nucleic acid probes and/or primers employed in the kit of the present invention desirably include nucleic acid probes and/or primers capable of detecting rs151290 with a genotype of C/A, rs163184 with a genotype of G/T, rs2237895 with a genotype of C/A, rs2237892 with a genotype of C/T, and/or rs2250402 with a genotype of C/A, and nucleic acid probes and/or primers that are capable of detecting rs2307027 with a genotype of C/T, rs3741872 with a genotype of C/T, rs574628 with a genotype of G/A, rs2233647 with a genotype of G/A, rs3785233 with a genotype of C/A, and/or rs2075931 with a genotype of A/G.

The above nucleic acid probes and/or primers can be separately (or in a mixed state, when possible) dissolved in water or a suitable buffer solution (such as TE buffer) to a suitable concentration (such as 1 to 50 μM at a 2X to 20X concentration), and be stored at about -20 degrees Celsius.

Based on the polymorphism detection method, the kit of the present invention may further comprise other components required to implement the particular method. For example, when a particular kit is for detecting a polymorphism by the TaqMan PCR method, the kit may further contain 10X PCR reaction buffer, 10X MgCl$_2$ aqueous solution, 10X dNTPs aqueous solution, Taq DNA polymerase (5 U/μL), and the like.

The present invention further relates to the prevention and/or treatment of type-2 diabetes through regulation (for example, suppression) of the expression and/or activity of the KCNQ1 gene and/or EIF2AK4 gene.

Substances interacting with (for example, promoting, suppressing, or stabilizing) expression of the KCNQ1 gene or the structure or function of the KCNQ1 protein are effective in the prevention or treatment of type-2 diabetes. Accordingly, the screening method of the present invention is a method of screening for an interacting substance, characterized by comprising the steps of subjecting a cell expressing the KCNQ1 gene (such as INS-1 cells, RIN cells, and other pancreatic β cells and cardiomuscular cells) to the action of a test substance; detecting a change in the expression of the KCNQ1 gene or a change in the function of the KCNQ1 protein; and selecting a substance interacting with the KCNQ1 gene or the KCNQ1 protein based on this change. The interactive substance obtained by screening is employed to prevent or treat type-2 diabetes as a preventive agent or treatment agent for type-2 diabetes. The same applies to substances interacting with the expression of the EIF2AK4 gene or the structure or function of the EIF2AK4 protein.

Examples of test substances are nucleic acids, proteins, peptides, non-peptide compounds, synthetic compounds, fermentation products, cell extracts, vegetable extracts, and animal tissue extracts. These substances may be novel or known substances.

The level of expression of the KCNQ1 (or EIF2AK4) gene can be measured as a transcription level by detecting the corresponding mRNA using nucleic acid capable of hybridizing under stringent conditions with nucleic acid coding for KCNQ1 (or EIF2AK4) (that is, nucleic acid containing the base sequence, or some portion thereof, coding for KCNQ1 (or EIF2AK4) (also referred to as "sense KCNQ1 (or EIF2AK4)" hereinafter) or a base sequence, or some portion thereof, complementary to the base sequence coding for KCNQ1 (or EIF2AK4) (antisense KCNQ1 (or EIF2AK4))). Alternately, the level of expression can be measured as a translation level by detecting a protein (peptide) using an anti-KCNQ1 (or EIF2AK4) antibody prepared by the usual known methods.

Accordingly, the present invention more specifically provides:

(1) a method of screening for substances interacting with the KCNQ1 (or EIF2AK4) gene or KCNQ1 (or EIF2AK4) protein, characterized by:

culturing a cell expressing the KCNQ1 (or EIF2AK4) gene in the presence and absence of a test substance; and measuring and comparing the quantities of mRNA coding for KCNQ1 (or EIF2AK4) under both conditions using sense or antisense KCNQ1 (or EIF2AK4);

(2) a method of screening for substances interacting with the KCNQ1 (or EIF2AK4) gene or KCNQ1 (or EIF2AK4) protein, characterized by:

culturing a cell expressing the KCNQ1 (or EIF2AK4) gene in the presence and absence of a test substance; and measuring and comparing the quantities of KCNQ1 (or EIF2AK4) protein (peptide) under both conditions using anti-KCNQ1 (or EIF2AK4) antibody; and (3) a method of screening for substances interacting with the KCNQ1 (or EIF2AK4) gene or KCNQ1 (or EIF2AK4) protein, characterized by:

culturing a cell expressing the KCNQ1 (or EIF2AK4) gene in the presence and absence of a test substance; and
measuring and comparing the function of the KCNQ1 (or EIF2AK4) protein under both conditions based on the change in channel activity by the patch clamp method.

For example, the quantity of mRNA or the quantity of protein (peptide) of KCNQ1 (or EIF2AK4) can be specifically measured in the following manner:

(i) A test substance is administered a certain period (30 minutes to 24 hours, desirably 30 minutes to 12 hours, preferably one hour to six hours) before, a certain period (30 minutes to 3 days, desirably one hour to two days, preferably one hour to 24 hours) after, or simultaneously with, the administration of a drug, physical stimulus, or the like to a healthy or diseased model non-human warm-blooded animal (such as a mouse, rat, rabbit, sheep, pig, cow, cat, dog, monkey, or bird) and heart muscle, pancreatic tissue, or the like is collected once a certain period has elapsed following the administration. The mRNA of KCNQ1 (or EIF2AK4) expressed in the cells expressing the KCNQ1 (or EIF2AK4) gene contained in the biosample can be quantified by, for example, extracting the mRNA from the cells or the like by the usual method and using a method such as the TaqMan assay or RT-PCR, or by known Northern blot analysis. Additionally, the quantity of KCNQ1 (or EIF2AK4) protein can be quantified by Western blot analysis or the various immunoassays set forth in detail below.
(ii) A transformant incorporating nucleic acid coding for KCNQ1 (or EIF2AK4) or a partial peptide thereof is prepared by the usual methods. A test substance is added to the medium in the course of culturing the transformant by the usual methods. Following culturing for a certain period, the quantity of mRNA or the quantity of protein (peptide) of KCNQ1 (or EIF2AK4) contained in the transformant can be quantified and analyzed.

Specific examples of methods of measuring the amount of KCNQ1 (or EIF2AK4) protein in the above screening methods are: (i) competitively reacting anti-KCNQ1 (or EIF2AK4) antibody, a test solution, and labeled KCNQ1 (or EIF2AK4) and detecting the labeled KCNQ1 (or EIF2AK4) that has bound to the antibody to quantify the KCNQ1 (or EIF2AK4) in the sample solution; and (ii) simultaneously or continuously reacting a sample solution, anti-KCNQ1 (or EIF2AK4) antibody insolubilized on a carrier, and separate labeled anti-KCNQ1 (or EIF2AK4) antibody followed by measuring the quantity (activity) of the labeling agent on the insoluble carrier to quantify the KCNQ1 (or EIF2AK4) protein in the sample solution.

In the quantification method of (ii) above, two antibodies that recognize different portions of the KCNQ1 (or EIF2AK4) protein are desirable. For example, it is possible to employ an antibody recognizing the N-terminus portion of the KCNQ1 (or EIF2AK4) protein and another antibody reacting with the C-terminus portion of the KCNQ1 (or EIF2AK4) protein.

A radioactive isotope, enzyme, fluorophore, or light-emitting substance, for example, can be employed as the labeling agent in the measurement method in which a labeled substance is employed. Examples of radioactive isotopes that can be employed are [$^{125}$I], [$^{131}$I], [$^{3}$H], and [$^{14}$C]. The enzyme is desirably a stable enzyme of great specific activity; β-galactosidase, β-glucosidase, alkaline phosphatase, peroxidase, malate dehydrogenase, and the like can be employed. Examples of fluorophores that can be employed are fluorescamine and fluorescein isothiocyanate. Examples of light-emitting substances that can be employed are luminol, luminol derivatives, luciferins, and lucigenin. A biotin-(strepto) avidin system can be employed to bind the antibody or antigen to the labeling agent.

When the KCNQ1 (or EIF2AK4) protein is localized within the cells, after suspending the cells in a suitable buffer solution, ultrasonic processing, freezing and thawing, or the like can be used to rupture the cells and obtain a sample solution in the form of a ruptured cell solution. When the KCNQ1 (or EIF2AK4) protein is secreted to the cell exterior, the cell culture supernatant can be employed as is as the sample solution. As needed, quantification can be conducted after separating and purifying the KCNQ1 (or EIF2AK4) protein from the ruptured cell solution or culture supernatant. Further, undamaged cells can be employed as sample to the extent that detection of the labeling agent is possible.

The method of quantifying the KCNQ1 (or EIF2AK4) protein employing anti-KCNQ1 (or EIF2AK4) antibody is not specifically limited. Any measurement method can be employed so long as the quantity of antibody, antigen, or antibody-antigen complex, based on the quantity of antigen in the sample solution, is detected by a chemical or physical means and this quantity is determined from a standard curve plotted with standard solutions containing known quantities of the antigen. For example, nephelometry, competitive methods, immunometric methods, and the sandwich method can be suitably employed. Use of the sandwich method, described further below, is desirable in terms of sensitivity and specificity.

Physical adsorption or the chemical bonding that is employed to insolubilize or immobilize common proteins, enzymes, or the like can be employed to insolubilize the antigen or antibody. Examples of carriers are insoluble polysaccharides such as agarose, dextran, and cellulose; synthetic resins such as polystyrene, polyacrylamide, and silicone; and glass.

In the sandwich method, insolubilized anti-KCNQ1 (or EIF2AK4) antibody is reacted with a sample solution (primary reaction); separate labeled anti-KCNQ1 (or EIF2AK4) antibody is reacted (secondary reaction); and the quantity or activity of the labeling agent on the insoluble carrier is measured to quantify the KCNQ1 (or EIF2AK4) in the sample solution. The primary and secondary reaction can be conducted in reverse order, simultaneously, or in staggered fashion. The labeling agent employed and the insolubilizing method can be based on those set forth above. In immunoassaying by the sandwich method, it is not necessary to employ a single antibody as the solid-phase antibody or labeled antibody; a mixture of two or more antibodies can be employed to enhance measurement sensitivity.

The anti-KCNQ1 (or EIF2AK4) antibody can be used by a system other than the sandwich method, such as the competitive method, immunometric method, or nephelometry.

In the competitive method, KCNQ1 (or EIF2AK4) protein in the sample solution and labeled KCNQ1 (or EIF2AK4) protein are competitively reacted with an antibody, unreacted labeled antigen (F) and labeled antigen (B) that has bound to the antibody are separated (B/F separation), and the labeled quantity of either B or F is measured to quantify the KCNQ1 (or EIF2AK4) protein in the sample solution. In this reaction method, a soluble antibody is employed as the antibody and polyethylene glycol, a secondary antibody relative to the above antibody (primary antibody), or the like is employed in a liquid phase method to conduct B/F separation; and a solid phase method, in which either a solid phase antibody is employed as the primary antibody (direct method) or a soluble primary antibody is employed and a solid-phase antibody is employed as the secondary antibody (indirect method), is employed.

In the immunometric method, KCNQ1 (or EIF2AK4) protein in the sample solution and solid-phase KCNQ1 (or EIF2AK4) protein are competitively reacted with a certain quantity of labeled antibody, followed by separating the solid phase and liquid phase. Alternatively, the KCNQ1 (or EIF2AK4) protein in the sample solution is reacted with an excess quantity of labeled antibody, followed by adding the solid-phase KCNQ1 (or EIF2AK4) protein so that the unreacted labeled antibody is bound to the solid phase, after which the solid phase and liquid phase are separated. Next, the labeled quantity of either one of the phases is measured to quantify the level of antigen in the sample solution.

In nephelometry, the quantity of insoluble precipitate produced as the result of an antigen-antibody reaction in a gel or solution is measured. Laser nephelometry utilizing laser scattering can be suitably employed even when there is only a trace quantity of KCNQ1 (or EIF2AK4) protein in the sample solution and only a small quantity of precipitate can be obtained.

When applying these immunological measurement methods as the quantification method in the present invention, no special conditions, operations, or other settings are required. It suffices to build a measurement system for KCNQ1 (or EIF2AK4) protein based on the usual technical considerations of a person having ordinary skill in the art with regard to the usual conditions and operation of these methods. Introductory works, authoritative works, and the like can be consulted for details on the general technical means employed in these methods.

For example, reference can be made to Hiroshi Irie, ed., Radioimmunoassays (Kodansha, released in 1974); Hiroshi Irie, ed., General Radioimmunoassays (Kodansha, released in 1979); Eiji Ishikawa et al., ed., Enzyme Immunoassay Methods (Igaku Shoin, released in 1978); Eiji Ishikawa et al., ed., Enzyme Immunoassay Methods (2nd Ed.) (Igaku Shoin, released in 1982); Eiji Ishikawa et al., ed., Enzyme Immunoassay Methods (3rd Ed.) (Igaku Shoin, released in 1987); Methods in Enzymology, Vol. 70 (Immunochemical Techniques (Part A)); Ibid., Vol. 73 (Immunochemical Techniques (Part B)); Ibid., Vol. 74 (Immunochemical Techniques (Part C)); Ibid., Vol. 84 (Immunochemical Techniques (Part D: Selected Immunoassays)); Ibid., Vol. 92 (Immunochemical Techniques (Part E: Monoclonal Antibodies and General Immunoassay Methods)); Ibid., Vol. 121 (Immunochemical Techniques (Part I: Hybridoma Technology and Monoclonal Antibodies)) (the above being published by Academic Press); and the like. The contents thereof are incorporated herein by reference.

As set forth above, by using anti-KCNQ1 (or EIF2AK4) antibody, it is possible to quantify with good sensitivity the amount of KCNQ1 (or EIF2AK4) protein produced in cells.

For example, change in the function of KCNQ1 protein can be specifically measured in the manner set forth below.

A cell strain in the form of CHO cells that have been caused to permanently express high levels of human KCNQ1 protein (hKvLQT1/hminK-CHO K1 recombinant cell line: see Millipore, catalog no. CYL3007; these contents are incorporated herein by reference) are employed. By means of the patch clamp method, changes in potential are electrically recorded to measure and compare the channel activity in the presence and in the absence of specific substances. This permits the measurement of changes in the function of KCNQ1 protein in the above cell strain. Further, high-throughput screening is possible using commercial equipment (FLIPR, IonWorks HT, PatchExpress, and the like). Still further, change in the function of EIF2AK4 protein can be detected by, for example, measuring the quantity of substrate that is phosphorylated by GCN2 kinase, an EIF2AK4 protein. As examples of such methods, generally known techniques such as ELISA, Western Blotting, and LC/MS/MS can be utilized with a $^{32}$P radioactive label and anti-phosphorylated tyrosine antibody, in addition to Pro-Q* diamond phosphoprotein gel stain (Invitrogen), which specifically dyes EIF2 protein $\alpha$ subunits that have been phosphorylated in polyacrylamide gel, and the like.

Changes in cellular function by substances interacting with a KCNQ1 (or EIF2AK4) gene or a KCNQ1 (or EIF2AK4) protein, which has been imparted a change in the expression of the KCNQ1 (or EIF2AK4) gene or a change in the function of the KCNQ1 (or EIF2AK4) protein can be measured by the following methods.

For example, for cells in which the expression of the KCNQ1 (or EIF2AK4) gene has been decreased by the presence of a drug, an insulin secretion assay is conducted by the usual method. Specifically, the drug is added to cells that have been cultured on a 24-well plate to decrease expression of the KCNQ1 (or EIF2AK4) gene. When 50 to 100 hours have elapsed following addition of the drug, pre-incubation is conducted, after which the cells are stimulated for 0.5 to 2 hours with high glucose (for example, 20 to 50 mM) and low glucose (for example, 1 to 10 mM). The supernatant is then recovered, the portion remaining in the cells is extracted with hydrochloric acid in ethanol, and the insulin contents of the supernatant and cells is measured by one of the usual methods (for example, ELISA). Insulin secretion can be denoted and compared as the ratio of the amount secreted to the total insulin level.

The present invention is described with greater specificity in the examples below. However, the present invention is not limited to the examples.

Example 1

1 Materials and methodology

(1) Samples

Three independent patient panels were assembled for multistage genome-wide screening. Panel 1 was comprised of 188 patients with type-2 diabetes. Panel 2 was comprised of 752 patients with type-2 diabetes and 752 non-diabetic patients. Panel 3 was comprised of 672 patients with type-2 diabetes and 672 non-diabetic patients. The criteria for test subject patients with type-2 diabetes was as follows: (1) age at initial onset: 40 to 55, (2) maximum BMI < 30 kg/m$^2$, (3) insulin treatment not begun during the three years following diagnosis, and (4) negative for anti-GAD (glutamate decarboxylase) antibody.

The criteria for the non-diabetic patients of panels 2 and 3 were: (1) age: 60 or more, (2) no past history of diagnosis of diabetes, and (3) hemoglobin A1 c < 5.6 percent. The type-2 diabetes patients of panel 3 and the non-diabetic patients of panels 2 and 3 were recruited from 11 facilities located throughout Japan. Genomic DNA was extracted from peripheral blood by the usual methods. Clinical information such as the BMI (body mass index), blood biochemistry (including plasma glucose and insulin level), and family history of type-2 diabetes were also obtained. The background characteristics of each panel are summarized in Table 1. The research protocols were approved by the ethics committee of each facility.

[Table 1]

Clinical characteristics of the various subject panels

| | Panel 1 | Panel 2 | | Panel 3 | |
|---|---|---|---|---|---|
| | Diabetic | Diabetic | Nondiabetic | Diabetic | Nondiabetic |
| $n$ | 187 | 752 | 752 | 672 | 672 |
| Male participants (%) | 61.9 | 57.8 | 46.3 | 57.9 | 38.7 |
| Age during study (years) | $63.0 \pm 8.9$ | $62.4 \pm 8.9$ | $69.2 \pm 6.9$ | $62.6 \pm 9.1$ | $70.2 \pm 6.4$ |
| Age at onset (years) | $48.1 \pm 4.8$ | $47.9 \pm 5.0$ | | $47.9 \pm 5.0$ | |
| BMI (kg/m$^2$) | $23.0 \pm 2.6$ | $23.3 \pm 3.0$ | $22.9 \pm 2.8$ | $23.1 \pm 3.0$ | $22.5 \pm 3.0$ |
| HbA$_{1c}$ (%) | $7.2 \pm 1.3$ | $7.5 \pm 1.5$ | $5.0 \pm 0.3$ | $7.6 \pm 1.5$ | $5.0 \pm 0.3$ |
| Data are average $\pm$ SD | | | | | |

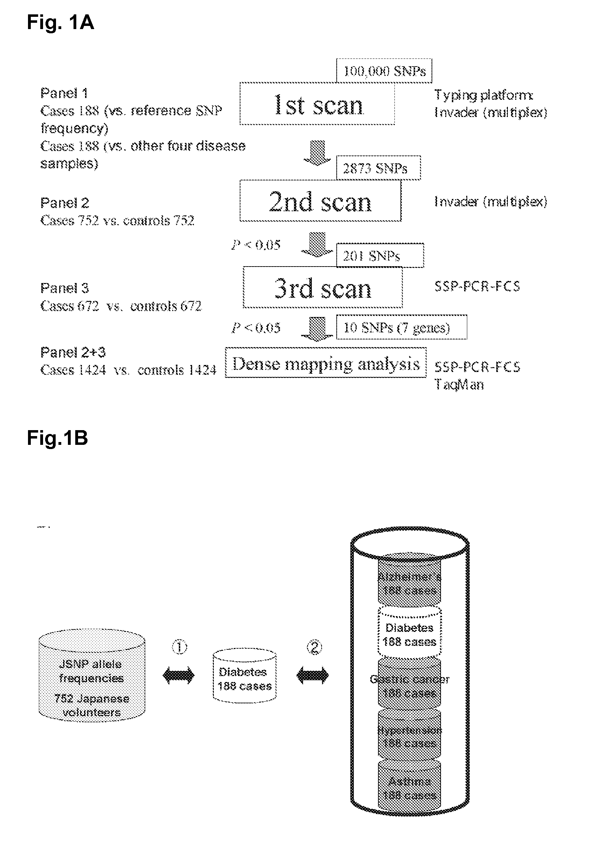

(2) Study design: Multistage genome-wide linkage analysis employing the Japanese SNP Database (Figs. 1A and 1 B)

Called the JSNP Genome Scan (JGS), multistage screening was planned by the usual methods. In the first screening, 188 patients with various diseases (diabetic panel 1) were genotyped for $10^5$ SNPs provided by the IMS-JST Japanese SNP Database Project. The following two sets of association analysis were conducted: 1) comparison of allele frequencies with reference data on the general Japanese population recorded in the above database; and 2) data on allele and/or genotype frequencies for patients obtained from the above project and a 752 person panel of non-diabetic patients from four other disease panels were compared (Fig. 1 B). Relative low allele frequencies (MAF) exceeded 10 percent in comparison with the database, and SNPs with genotypes of OR>1.5 or alleles of OR> 1.3 were selected for a second screening. When SNPs exhibiting positive association that were numerous in the same gene were in strong linkage disequilibrium (LD), to avoid redundancy, just one SNP was selected for subsequent screening. Subsequently, 2,880 SNPs of various diseases (2,873 for diabetes) were selected by p value.

In the second screening, an independent type-2 diabetic patient/non-diabetic patient panel (panel 2) was analyzed. Following a data quality check, data on 2,827 suitable SNPs were generated. SNPs exhibiting an association with diabetes (P < 0.05) based on allele data were selected as targets for a third screening.

In the third screening, a separate panel (panel 3) was genotyped. SNPs that were positive (P < 0.05) in the third screening were analyzed in combination with the results of panel 2 (panels 2 and 3: 1,424 diabetic patients and 1,424 non-diabetic patients).

Subsequently, dense SNP mapping of the KCNQ1 gene was conducted. Initially, further SNPs were taken from the NCBI SNP Database to obtain an interval of about 10 kb and genotyped in panels 2 and 3 along with the positive SNPs originally included in the present genome scan. Genes including all of the exons and presumed promoter regions (4 kbp upstream from the transcription starting site) were resequenced for 24 Japanese subjects to identify widespread genetic variants among the Japanese. The peripheral regions of positive SNPs were resequenced over 47 kbp (intron 15). Some of the SNPs thus identified were subsequently genotyped in panels 2 and 3.

(3) Genotyping methods

In the first and second screenings, genotyping was conducted by multiplex polymerase chain reaction (PCR)-based invader assay (Third Wave Technologies, Madison, Wisconsin, USA) according to the usual methods. In the third screening and dense mapping, a DOP-PCR template that had been amplified on a genomic scale was genotyped by sequence-specific primer (SSP)-PCR by fluorescence correlation spectroscopy (FCS). As a result, the SNPs contained in both the second screening and dense mapping were again genotyped by the SSP-PCR-FCS method for panel 2. A number of SNPs were genotyped by real-time PCR using TaqMan probe (Applied Biosystems, Foster City, CA).

(4) Tissue expression analysis of genes by real-time PCR

The islets of Langerhans were isolated from five female 12-week-old C57BL6 or KK-Ay mice that had fasted for 14 hours and complete RNA was extracted with an RNeasy kit (Qiagen, Hilden, German). Employing TATA binding protein as an internal standard, the expression level of murine Kcnql mRNA was analyzed by real-time RT-PCR using TaqMan gene expression assay (Applied Biosystems) at AB17900HT. The results exhibited a double change level relative to unafflicted BL6 mice.

(5) Statistical analysis

As set forth above, in the first screening, two sets of evaluation were conducted for type-2 diabetic patients and non-diabetic patients. Four groups of non-diabetic patients were employed and the data were statistically analyzed in 2x2 and 2x3 contingency tables for comparison. For comparison with the frequency in the general population, for which genotype data could not be used, just allele data were analyzed in 2x2 contingency tables.

In the second and third screenings and in the dense mapping, the allele data in the 2x2 contingency tables were analyzed by an $x^2$ test. Linkage disequilibrium and haplotype analysis (analysis of the lineup of genes derived from a single parent when multiple alleles are divided into genes inherited from individual parents) were conducted with Haploview 3.31. Haplotype frequency evaluation was also calculated with PHASE. Statistical analysis of clinical characteristics was conducted with StatView Version 5.1 (SAS, Cary, NC). Whenever necessary, the data were log converted. ANCOVA was used to determine p values adjusted for age, sex, and BMI. $p < 0.05$ was considered statistically significant.

2 Results

(1) Multistage association analysis of diabetes

Of the $10^5$ SNPs that were genotyped for the JGS by multiplex PCR-based invader assay in the first screening, the data of 82,343 SNPs of the autosome were determined to have passed the genotyping quality check for 187 type-2 diabetes patients. For one subject, no genotype calls were made. Based on two combined correlation analyses, 2,873 SNPs were selected as targets for the second screening.

In the second screening, 38 SNPs produced no results for any of the persons tested in panel 2. Further, five SNPs and three SNPs failed for either type-2 diabetic patients or non-diabetic patients in the multiplex PCR-based invader assay. The call rate for the remaining 2,827 SNPs was 0.993. Subsequently, 201 positive SNPs (P < 0.05) were selected for the third screening.

In the third screening, one SNP could not be genotyped at all in panel 3. The call rate for the other 200 SNPs was 0.990. Ten SNPs of seven genes had p values of lower than 0.05 (Table 3). The most pronounced correlation, shown as p value of 3.4 x $10^{-6}$, was seen in rs2237895 (IMS-JST018602) in intron 15 of the KCNQ1 gene. Another two SNPs (IMS-JS018590 and 018601, corresponding to rs151290 and rs16184, respectively), also localized in the same intron, exhibited p values of $1.1 \times 10^{-4}$ and 0.0021, respectively. Panel 2 was again genotyped for these 10 SNPs by the FCS-SSP-PCR method. The consistency rate with the invader method (returning to the second screening) was 0.992. The combined results for the two panels (panels 2+3) were again analyzed for these 10 SNPs, and all of the SNPs, including the three in the KCNQ1 gene, exhibited relatively low p values (Tables 2A and 2B). In Tables 2A and 2B, an asterisk (*) denotes a dangerous allele, Chr denotes a chromosome number, DM denotes a type-2 diabetic patient, and NC denotes a non-diabetic patient. For example, "DM12 of rs151290" indicates a type-2 diabetic patient who was a heterozygote of rs151290 genotype A/C. As set forth above, the p value was calculated by comparing allele frequencies between the type-2 diabetic patient group and the non-diabetic patient group and conducting $\times^2$ verification. OR denotes the odds ratio of a particular dangerous allele.

[Table 2A]

| ISNP ID | A1 | A2 | dbSNP ID (n#) | Chr | Gene | Panel 2 | | | | | | | Panel 3 | | | | | | |
|---|---|---|---|---|---|---|---|---|---|---|---|---|---|---|---|---|---|---|---|
| | | | | | | DM11 | DM12 | DM22 | NC11 | NC12 | NC22 | P value | DM11 | DM12 | DM22 | NC11 | NC12 | NC23 | P value |
| MS-IST018590 | A | C* | 151290 | 11 | *KCNQI* | 114 | 342 | 194 | 163 | 351 | 236 | $7.4\times10^{-7}$ | 101 | 314 | 249 | 136 | 346 | 185 | $1.1\times10^{-4}$ |
| IMS-IST018601 | T | G* | 163184 | 11 | *KCNQI* | 194 | 378 | 172 | 245 | 338 | 156 | 00064 | 174 | 346 | 145 | 219 | 328 | 112 | 0.0021 |
| IMS-IST018602 | A | C* | 2237895 | 11 | *KCNQI* | 153 | 361 | 138 | 348 | 302 | 95 | 215 | 215 | 344 | 106 | 294 | 298 | 70 | $3.4\times10^{-5}$ |
| IMS-IST064933 | A | C* | 2250402 | 15 | *EIF1AK4* | 440 | 253 | 52 | 470 | 244 | 33 | 0.035 | 348 | 258 | 39 | 413 | 206 | 33 | 0.0018 |
| IMS-IST064834 | T | C* | | 12 | *KRF4* | 476 | 238 | 31 | 515 | 214 | 21 | 0.031 | 413 | 229 | 26 | 464 | 182 | 17 | 0.0017 |
| IMS-IST092942 | T | C* | 3741872 | 12 | *FAM60A* | 375 | 321 | 54 | 414 | 276 | 40 | 0.0024 | 354 | 251 | 62 | 388 | 233 | 38 | 0.0060 |
| IMS-IST144420 | A | G* | 574628 | 20 | *ANGPT4* | 91 | 345 | 314 | 109 | 362 | 276 | 0.017 | 87 | 298 | 279 | 117 | 321 | 232 | 0.0018 |
| IMS-IST010811 | A | G* | 2233647 | 6 | *5PDEF* | 13 | 146 | 578 | 8 | 194 | 542 | 0.047 | 11 | 125 | 526 | 18 | 148 | 498 | 0.033 |
| IMS-IST141777 | A | C* | 3785233 | 16 | *AIBPI* | 502 | 216 | 33 | 533 | 201 | 17 | 0.023 | 429 | 221 | 26 | 469 | 182 | 19 | 0.039 |
| IMS-IST003807 | A* | G | 2075931 | 1 | | 349 | 324 | 75 | 311 | 336 | 93 | 0.038 | 308 | 293 | 67 | 266 | 330 | 73 | 0.048 |

[Table 2B]

| | Panel 2+3 | | | | | | | Panel 2+3 | | | | | | | |
|---|---|---|---|---|---|---|---|---|---|---|---|---|---|---|---|
| DM11 | DM12 | DM22 | NC11 | NC12 | NC22 | $P$ value | DM A1 | DM A2 | NC A1 | NC A2 | OR | 95% | CI | HWE-DM | HWE-NC |
| 215 | 656 | 543 | 299 | 691 | 421 | $3.0 \times 10^{-8}$ | 0.38 | 0.62 | 0.46 | 0.54 | 1.35 | 1.21 | 1.50 | 0.47 | 0.62 |
| 368 | 724 | 317 | 464 | 666 | 262 | $4.3 \times 10^{-5}$ | 0.52 | 0.48 | 0.57 | 0.43 | 1.25 | 1.12 | 1.38 | 0.28 | 3.40 |
| 468 | 705 | 244 | 642 | 600 | 165 | $2.2 \times 10^{-22}$ | 0.58 | 0.42 | 6.67 | 0.33 | 1.47 | 1.32 | 1.64 | 0.44 | 0.17 |
| 788 | 511 | 91 | 883 | 450 | 66 | $2.4 \times 10^{4}$ | 0.75 | 0.25 | 0.79 | 0.21 | 1.26 | 1.12 | 1.43 | 0.51 | 0.37 |
| 889 | 467 | 57 | 979 | 396 | 38 | $2.0 \times 10^{-4}$ | 0.79 | 0.21 | 0.83 | 0.17 | 1.29 | 1.13 | 1.48 | 0.66 | 0.79 |
| 729 | 572 | 116 | 822 | 509 | 78 | $4.3 \times 10^{-5}$ | 0.72 | 0.28 | 0.76 | 0.24 | 1.28 | 1.14 | 1.44 | 0.80 | 0.95 |
| 178 | 643 | 593 | 226 | 683 | 508 | $2.4 \times 10^{-8}$ | 0.35 | 0.65 | 0.40 | 0.60 | 1.22 | 1.10 | 1.36 | 0.86 | 0.39 |
| 24 | 271 | 1104 | 26 | 342 | 1041 | 0.0037 | 0.11 | 0.89 | 0.14 | 0.86 | 0.14 | 1.08 | 1.48 | 0.12 | 0.73 |
| 931 | 437 | 53 | 1002 | 383 | 36 | 0.0022 | 0.81 | 0.19 | 0.84 | 0.16 | 1.26 | 1.08 | 1.42 | 0.85 | 0.93 |
| 657 | 617 | 142 | 577 | 666 | 166 | 0.0042 | 0.68 | 0.32 | 0.65 | 0.35 | 1.18 | 1.05 | 1.3 | 0.87 | 0.21 |

(2) Dense mapping and KCNQ1 association analysis

KCNQ1 gene [a] exhibits the strongest correlation to type-2 diabetes patients, [b] is the only gene for which positive results straddle multiple SNPs, and [c] is localized to chromosome 11 p15.5. It is contained in a candidate region exhibiting a linkage to diabetes that has been proposed by two independent reports analyzing Japanese nationals who have contracted diabetes. (See Mori, Y. et al., (2002), Diabetes 51, 1247-1255; and Nawata, H. et al., (2004), J. Hum. Genet. 49, 629-634, the contents of which are incorporated herein by reference). Neither of these reports provides a precise mapping, but the greatest LOD scores of this region were 3.08 and 2.89, respectively. As set forth above, the KCNQ1 gene is an important gene associated with the onset of type-2 diabetes, which has now been discovered for the first time. Accordingly, the KCNQ1 gene was further analyzed following the third screening.

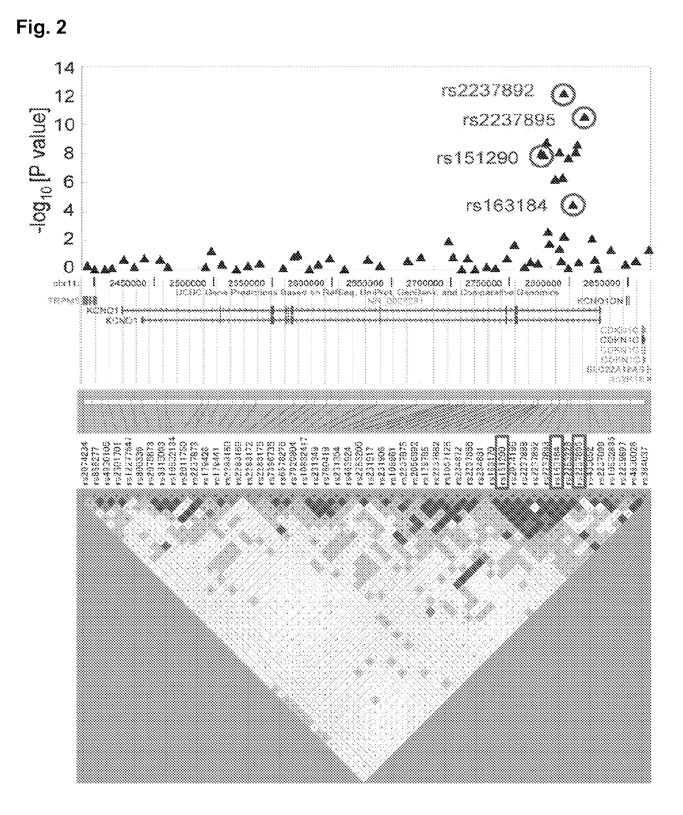

As shown in the lower portion of Fig. 2, the three SNPs (rs151290, rs163184, and rs2237895) that has passed the third screening were all mutually in "suitable" LD. rs223789, which exhibited the lowest p value, had a D' and r2 value with rs151290 of 0.544 and 0.124, and a D' and r2 value with rs163184 of 0.83 and 0.455, respectively. Forty-nine SNPs were picked up from the NCBI SNP Database and were genotyped in panels 2 and 3 with these three SNPs, which were positive in the original scan (Fig. 2). Among the 52 SNPs, rs2237892 was localized to intron 15 and exhibited the strongest association with type-2 diabetes (p=6.7 x $10^{-13}$; panels 2 and 3) as shown in the upper portion of Fig. 2. The OR was 1.49 (95 percent CI: 1.34-1.66). The D' and R2 values with rs2237895 and rs 2237892 were 0.952 and 0.297, respectively.

Two hundred and twelve variants, including three synonymous substitutions and two non-synonymous substitutions (P448R and G643R) were identified by resequencing genetic regions of 24 Japanese subjects. Ten SNPs with minor allele frequencies (MAFs) exceeding 10 percent were selected in the 35.6 kbp region between rs151290 and rs2237895 and were genotyped in panels 2 and 3. In total, 18 SNPs having average intervals of 2 kbp were genotyped between rs151290 and rs2237895. Two non-synonymous substitution polymorphisms were also genotyped. None of these SNPs exhibited a stronger association with diabetes than rs2237892 (Fig. 2).

(3) The effect of KCNQ1 variant on clinical parameters related to glucose homeostasis

Haplotypes were constructed from rs2237892 (C/T; risk allele = C) and rs2237895 (A/C; risk allele = C), which exhibited the greatest and second greatest association among all the SNPs that were genotyped. The T-A haplotype (p = 3.2 x $10^{-13}$) was discovered to have the most marked association with disease in panels 2+3. Since the above p value was not greater than the p value for rs2237892 alone (P = 6.7 x $10^{-13}$), the possibility of a causative variant in the gene was high. A detailed analysis was conducted employing rs2237892 alone. Next, the effect of the risk allele of rs2237892 on the form of clinical expression was examined. No association between the above SNPs and clinical parameters such as BMI, the age of onset of diabetes, and the level of insulin resistance (no data) was discovered among the 1,424 diabetic patients in panels 2+3.

Among 948 non-diabetic patients in panels 2+3 for whom fasting blood sugar levels and insulin levels were available, homozygotes for the risk allele of rs2237892 (CC, n=353) exhibited markedly lower homeostasis model assessment β *cell function* index (HOMA-β than other genotypes (CT and TT, n=595). The HOMA-β index were 81.7 $\pm$ 57.9 and 94.3 $\pm$ 84.3, respectively (P = 0.0024, with the P value after adjustment for sex, age, and BMI being 0.02). These results were not confirmed in diabetic patients, but this suggests that the risk allele of KCNQ1 contributes to the rate of onset of diabetes by blocking the secretion of insulin.

Example 2

The expression of Kcnq1 in the islets of Langerhans in a type-2 diabetes mouse model was examined by reverse transcription and real-time PCR. The level of expression of Kcnq1 mRNA in the islets of Langerhans of 12-week-old diabetic KK-Ay mice clearly exhibiting both hyperglycemia and hyperinsulinemia markedly increased by 1.6 fold relative to C57BL6 control mice (P = 0.0004).

Example 3

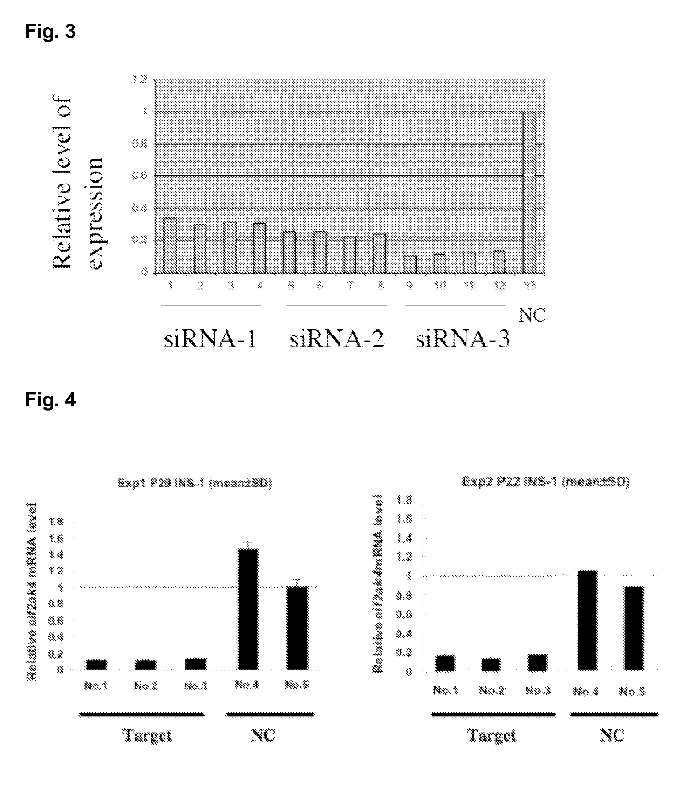

A screening system for substances changing the expression of the KCNQ1 gene was examined. Pancreatic β cell strain INS-1 cells (rat) were cultured in RPMI medium (10 percent FCS added). The Kcnq1 gene (rat KCNQ1 gene) siRNAs shown in SEQ. ID. NOS. 1 to 3 in the SEQUENCE LISTING were designed and transfected using a transfection drug (Lipofectamine 2000). Forty-eight to 72 hours later, total RNA was recovered and the expression level of the Kcnq1 gene was quantified with TaqMan assay. As a result, all three of the siRNAs administered to the pancreatic β cell strain INS-1 cells were determined to reduce expression by about 70 percent as an mRNA level relative to negative controls that were not administered the siRNAs, that is, a drop to about 30 percent in expression level was confirmed (Fig. 3).

Example 4

SNPs of the KCNQ1 gene of a new panel 4 (1,000 type-2 diabetes patients and 1,000 non-diabetes patients) distinct from panels 1 to 3 were measured in the same manner as in Example 1, and association analysis was conducted based on the data obtained. In the same manner as the analysis results in Example 1, all the SNPs of registry numbers rs151290, rs2237895, and rs2237892 in the NCBI SNP Database in the KCNQ1 gene exhibited strong association in type-2 diabetes patients (Table 3). Accordingly, the analysis results of panel 4 demonstrated the extremely high reproducibility of the analysis results of Example 1.
[Table 3]

| snp | A1 | A2 | DM11 | DM12 | DM22 | NC11 | NC12 | NC22 | DM_F_A1 | DM_F_A2 | NC_F_A1 | NC_F_A2 | A_P |
|---|---|---|---|---|---|---|---|---|---|---|---|---|---|
| rs151290 | A | C | 153 | 475 | 369 | 221 | 495 | 284 | 0.39 | 0.61 | 0.47 | 0.53 | 9.4E-07 |
| rs2074196 | T | G | 150 | 466 | 383 | 217 | 478 | 304 | 0.38 | 0.62 | 0.46 | 0.54 | 2.9E-06 |
| rs2237892 | C | T | 454 | 440 | 105 | 344 | 480 | 176 | 0.67 | 0.33 | 0.58 | 0.42 | 2.9E-09 |
| rs2237895 | A | C | 358 | 486 | 153 | 430 | 466 | 104 | 0.60 | 0.40 | 0.66 | 0.34 | 7.9E-05 |

Example 5

In the same manner as in Example 4, the SNP of registry number rs2250402 in the NCBI SNP Database in the EIF2AK4 gene in panel 4 (1,000 type-2 diabetes patients and 1,000 non-diabetes patients) was measured and association analysis was conducted on the data obtained in combination with the measurement data of panels 2 and 3. The result was a p value of $3.4 \times 10^{-5}$, with this SNP exhibiting strong association in type-2 diabetic patients.

Example 6

A system for screening substances changing the expression of the EIF2AK4 gene was examined in the same manner as in Example 3. The Eif2ak4 gene (rat EIF2AK4 gene) siRNAs shown in SEQ. ID. NOS. 4 to 6 in the SEQUENCE LISTING were designed and transfected using a transfection drug (Lipofectamine 2000) into two successive generations of pancreatic β cell strain INS-1 cells. Forty-eight to 72 hours later, total RNA was recovered and the expression level of the EIF2AK4 gene was quantified with TaqMan assay. As a result, all three of the siRNAs administered to the pancreatic β cell strain INS-1 cells were determined to reduce expression by about 70 to 90 percent as an mRNA level relative to negative controls that were not administered the siRNAs (Fig. 4).

**[Brief Description of the Drawings]**

[Fig. 1A] A schematic chart of multistage genome-wide linkage analysis using the Japanese SNP Database.
[Fig. 1B] A schematic chart of the two sets of association analysis in the first screening.
[Fig. 2] Fig. 2 shows dense mapping of the KCNQ1 gene.
[Fig. 3] Fig. 3 shows the level of expression of Kcnq1 following siRNA administration.
[Fig. 4] Fig. 4 shows the level of expression of Eif2ak4 following siRNA administration.

**Claims**

1. A method of testing for genetic susceptibility of a subject to type-2 diabetes, comprising detecting one or more polymorphisms present in the KCNQ1 gene in a DNA-containing sample collected from the subject.

2. A method of testing for genetic susceptibility to type-2 diabetes of a subject, comprising testing for one or more polymorphisms selected from the group consisting of polymorphisms present in the KCNQ1 gene with a higher frequency of one of the alleles among some group of type-2 diabetes patients than among some group of persons who have not contracted type-2 diabetes in a DNA-containing sample collected from the subject.

3. The method of Claim 1 or 2, wherein said polymorphisms are comprised of polymorphisms that are exhibited at registry numbers rs151290, rs163184, rs2237895, and rs2237892 in the NCBI SNP Database, and polymorphisms in a state of linkage disequilibrium with these polymorphisms such that the linkage disequilibrium coefficient D' is 0.9 or greater.

4. The method of Claim 3, wherein a high genetic susceptibility to type-2 diabetes is determined to be present when the rs151290 genotype is C, the rs163184 genotype is G, the rs2237895 genotype is C, and/or the rs2237892 genotype is C.

5. A method of testing for genetic susceptibility of a subject to type-2 diabetes, comprising detecting one or more polymorphisms present in the EIF2AK4 gene in a DNA-containing sample collected from the subject.

6. A method of testing for genetic susceptibility of a subject to type-2 diabetes, comprising testing for one or more polymorphisms selected from the group consisting of polymorphisms present in the EIF2AK4 gene with a higher frequency of one of the alleles among some group of type-2 diabetes patients than among some group of persons who have not contracted type-2 diabetes in a DNA-containing sample collected from the subject.

7. The method of Claim 5 or 6, wherein said polymorphisms are comprised of the polymorphism that is exhibited at registry number rs2250402 in the NCBI SNP Database and polymorphisms in a state of linkage disequilibrium with this polymorphism such that the linkage disequilibrium coefficient D' is 0.9 or greater.

8. The method of Claim 7, wherein a high genetic susceptibility to type-2 diabetes is determined to be present when the rs2250402 genotype is C.

9. The method of any of Claims 1 to 8, further comprising the detection of one or more polymorphisms selected from the group consisting of polymorphism exhibited at registry numbers rs2307027, rs3741872, rs574628, rs2233647, rs3785233, and rs2075931 in the NCBI SNP Database and polymorphisms in a state of linkage disequilibrium with these polymorphisms such that the linkage disequilibrium coefficient D' is 0.9 or greater with a higher frequency of one of the alleles among some group of type-2 diabetes patients than among some group of persons who have not contracted type-2 diabetes.

10. The method of Claim 9, wherein a high genetic susceptibility to type-2 diabetes is determined to be present when the rs2307027 genotype is C, the rs3741872 genotype is C, the rs574628 genotype is G, the ras2233647 genotype is G, the rs3785233 genotype is C, and/orthe rs2075931 genotype is A.

11. A kit for testing for genetic susceptibility to type-2 diabetes, comprising one or more nucleic acid probes and/or primers capable of detecting one or more polymorphisms present in the KCNQ1 gene and/or the EIF2AK4 gene.

12. A kit for testing for genetic susceptibility to type-2 diabetes, comprising one or more nucleic acid probes and/or primers capable of detecting one or more polymorphisms selected from the group consisting of polymorphisms exhibited at registry numbers rs151290, rs163184, rs2237895, rs2237892 and rs2250402 in the NCBI SNP Database, and polymorphisms in a state of linkage disequilibrium with these polymorphisms such that the linkage disequilibrium coefficient D' is 0.9 or greater.

13. The kit of Claim 12, wherein said nucleic acid probe and/or primer is a nucleic acid probe and/or primer that is capable of detecting rs151290 with a genotype of C/A, rs163184 with a genotype of G/T, rs2237895 with a genotype of C/A, rs2237892 with a genotype of C/T, and/or rs2250402 with a genotype of C/A.

**14.** The kit of any one of Claims 11 to 13, further comprising a nucleic acid probe and/or primer that is capable of detecting one or more polymorphisms selected from the group consisting of polymorphisms exhibited at registry numbers rs2307027, rs3741872, rs574628, rs2233647, rs3785233, and rs2075931 in the NCBI SNP Database and polymorphisms in a state of linkage disequilibrium with these polymorphisms such that the linkage disequilibrium coefficient D' is 0.9 or greater.

**15.** The kit of Claim 14, wherein said nucleic acid probe and/or primer is a nucleic acid probe and/or primer that is capable of detecting rs2307027 with a genotype of C/T, rs3741872 with a genotype of C/T, rs574628 with a genotype of G/A, rs2233647 with a genotype of G/A, rs3785233 with a genotype of C/A, and/or rs2075931 with a genotype of A/G.

**16.** A method for screening for an interacting substance, comprising the steps of subjecting a cell expressing the KCNQ1 gene to a test substance; detecting a change in the expression of the KCNQ1 gene or a change in the function of the KCNQ1 protein; and selecting a substance interacting with the KCNQ1 gene or the KCNQ1 protein based on this change.

**17.** A method for screening for an interacting substance , comprising the steps of subjecting a cell expressing the EIF2AK4 gene to a test substance; detecting a change in the expression of the EIF2AK4 gene or a change in the function of the EIF2AK4 protein; and selecting a substance interacting with the EIF2AK4 gene or the EIF2AK4 protein based on this change.

**18.** The method of Claim 16 or 17, wherein said interactive substance is one that is employed to prevent or treat type-2 diabetes.

## Fig. 1A

Panel 1
Cases 188 (vs. reference SNP frequency)
Cases 188 (vs. other four disease samples)

Panel 2
Cases 752 vs. controls 752

Panel 3
Cases 672 vs. controls 672

Panel 2+3
Cases 1424 vs. controls 1424

100,000 SNPs

1st scan — Typing platform: Invader (multiplex)

2873 SNPs

2nd scan — Invader (multiplex)

$P < 0.05$

201 SNPs

3rd scan — SSP-PCR-FCS

$P < 0.05$

10 SNPs (7 genes)

Dense mapping analysis — SSP-PCR-FCS TaqMan

## Fig.1B

## Fig. 2

**Fig. 3**

**Fig. 4**

## INTERNATIONAL SEARCH REPORT

| | International application No. |
|---|---|
| | PCT/JP2008/072786 |

**A. CLASSIFICATION OF SUBJECT MATTER**
*C12Q1/68*(2006.01)i, *C12N15/09*(2006.01)i, *C12Q1/02*(2006.01)i, *G01N33/15*(2006.01)i, *G01N33/50*(2006.01)i

According to International Patent Classification (IPC) or to both national classification and IPC

**B. FIELDS SEARCHED**

Minimum documentation searched (classification system followed by classification symbols)
C12Q1/68, C12N15/09, C12Q1/02, G01N33/15, G01N33/50

Documentation searched other than minimum documentation to the extent that such documents are included in the fields searched
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
BIOSIS/CAplus/MEDLINE/WPIDS(STN), JSTPlus/JMEDPlus(JDreamII), PubMed

**C. DOCUMENTS CONSIDERED TO BE RELEVANT**

| Category* | Citation of document, with indication, where appropriate, of the relevant passages | Relevant to claim No. |
|---|---|---|
| P,X | UNOKI, H. et al., 'SNPs in KCNQ1 are associated with susceptibility to type 2 diabetes in East Asian and European populations', Nat. Genet., 2008.09, Vol. 40, No. 9, pp.1098-1102 | 1-4,9-15 |
| P,X | YASUDA, K. et al., 'Variants in KCNQ1 are associated with susceptibility to type 2 diabetes mellitus', Nat. Genet., 2008.09, Vol. 40, No. 9, pp.1092-1097 | 1-4,9-15 |
| P,X | Kazuki YASUDA, "Kaimei Saretsutsu aru Tonyobyo Idenshi 04 2 Gata Tonyobyo to Idenshi 2)-Genome-wide Sokan Kaiseki ni yoru Approach", Medical Bio, 2008.11, Vol.5, No.6, pages 48 to 53 | 1-4,9-15 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 March, 2009 (04.03.09) | 17 March, 2009 (17.03.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

<div align="center">

**INTERNATIONAL SEARCH REPORT**

</div>

International application No.

PCT/JP2008/072786

C (Continuation).   DOCUMENTS CONSIDERED TO BE RELEVANT

| Category* | Citation of document, with indication, where appropriate, of the relevant passages | Relevant to claim No. |
|---|---|---|
| Y | ULLRICH, S., et. al, 'Effects of IKs channel inhibitors in insulin-secreting INS-1 cells', Eur. J. Physiol., 2005, Vol. 451, pp.428-436 | 1-4,9-15 |
| Y | MORI, Y. et al., 'Genome-Wide Search for Type 2 Diabetes in Japanese Affected Sib-Pairs Confirms Susceptibility Genes on 3q, 15q, and 20q and Identifies Two New Candidate Loci on 7p and 11p', Diabetes, 2002, Vol. 51, pp.1247-1255 | 1-4,9-15 |
| Y | NAWATA, H. et al., 'Genome-wide linkage analysis of type 2 diabetes mellitus reconfirms the susceptibility locus on 11p13-p12 in Japanese', J. Hum. Genet., 2004, Vol. 49, pp.629-634 | 1-4,9-15 |
| Y | HORIKAWA, Y., et al., 'Genetic variation in the gene encoding calpain-10 is associated with type 2 diabetes mellitus', Nat. Genet., 2000, Vol. 26, No. 2, pp.163-175 | 1-4,9-15 |
| A | FLOREZ, J. C., et al., 'TCF7L2 Polymorphisms and Progression to Diabetes in the Diabetes Prevention Program', N. Engl. J. Med., 2006, Vol. 355, No. 3, pp.241-250 | 1-4,9-15 |

Form PCT/ISA/210 (continuation of second sheet) (April 2007)

## INTERNATIONAL SEARCH REPORT

International application No.

PCT/JP2008/072786

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:
It is considered that the inventions of claims 1-15 include the following three inventions: the inventions of claims 1-4 and parts of the inventions of claims 9-15 which relate to KCNQ1 gene; the inventions of claims 5-8 and parts of the inventions of claims 9-15 which relate to EIF2AK4 gene; and inventions of claims 16-18.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
1-4, 9-15

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007325366 A
- WO 03023063 A

**Non-patent literature cited in the description**

- **Horikawa, Y. et al.** *Nat. Genet.,* 2000, vol. 26, 163-175
- **Weedon, M. N. et al.** *Diabetes,* 2004, vol. 53, 3002-3006
- **Mori, Y. et al.** *Diabetes,* 2002, vol. 51, 1247-1255
- **Nawata, H. et al.** *J. Hum. Genet.,* 2004, vol. 49, 629-634
- **Vincent.** *Ann. Med.,* February 1998, vol. 30 (1), 58-65
- **Howard C. Towlel.** *Cell Metabolism,* February 2007, vol. 5
- **Wallace et al.** *Proc. Natl. Acad. Sci. USA,* 1983, vol. 80, 278-282
- Radioimmunoassays. Kodansha, 1974
- General Radioimmunoassays. Kodansha, 1979
- Enzyme Immunoassay Methods. Igaku Shoin, 1978
- Enzyme Immunoassay Methods. Igaku Shoin, 1982
- Enzyme Immunoassay Methods. Igaku Shoin, 1987
- Immunochemical Techniques (Part A). Methods in Enzymology. vol. 70
- Immunochemical Techniques (Part B). METHODS IN ENZYMOLOGY. vol. 73
- Immunochemical Techniques (Part C). METHODS IN ENZYMOLOGY. vol. 74
- Immunochemical Techniques (Part D: Selected Immunoassays). METHODS IN ENZYMOLOGY. vol. 84
- Immunochemical Techniques (Part E: Monoclonal Antibodies and General Immunoassay Methods). METHODS IN ENZYMOLOGY. vol. 92
- Immunochemical Techniques (Part I: Hybridoma Technology and Monoclonal Antibodies). METHODS IN ENZYMOLOGY. Academic Press, vol. 121